# FASCICULE DE BREVET EUROPEEN

(11) **EP 1 626 041 B1**
(45) Date de publication et mention de la délivrance du brevet: **13.07.2016**
(21) Numéro de dépôt: 05023466.5
(22) Date de dépôt: 03.02.1999
(51) Int. Cl.: C07C 317/04, C07D 339/06, C07D 311/82, C07C 317/12, C08G 61/02, C08F 232/04, H01M 6/16

(54) **Nouveaux matériaux utiles en tant que solutés électrolytiques**
Neue Materialien verwendbar als elektrolytische Solubilisate
New materials useful as electolytic solutes

(30) Priorité: 03.02.1998 CA 2228801; 18.12.1998 CA 2256945
(43) Date de publication de la demande: 15.02.2006
(62) Demande divisionnaire de: 99903554.6
(73) Titulaire: ACEP Inc., Montréal, Quebec H2Z 1A4 (CA); Centre National de la Recherche Scientifique, 75794 Paris Cedex 16 (FR); Université de Montréal, Montréal, Québec H3C 3J7 (CA)
(72) Inventeur: Michot, Christophe, 38000 Grenoble (FR); Armand, Michel, Montréal Québec H3T 1N2 (CA); Gauthier, Michel, La Prairie Québec J5R 1E6 (CA); Ravet, Nathalie, Montréal Québec H3T 1T9 (CA)
(74) Mandataire: Goulard, Sophie

(56) Documents cités:
- WO-A1-97/02252
- US-A- 5 538 812
- P.L. DHINGRA, ET AL.: "Chemistry of imidobis(sulphuryl fluoride): Part II - Behaviour of electrolytes in imidobis(sulphuryl fluoride)" INDIAN JOURNAL OF CHEMISTRY, SECTION A, vol. 24A, no. 6, juin 1985 (1985-06), pages 472-475, XP002104844 NEW DEHLI, IN ISSN: 0376-4710
- H.W. ROESKY, ET AL.: "Darstellung und Untersuchung von Fluorsulfurylverbindungen" CHEMISCHE BERICHTE, vol. 101, no. 1, janvier 1968 (1968-01), pages 162-173, XP002104843 VERLAG CHEMIE, WEINHEIM, DE ISSN: 0009-2940
- H.W. ROESKY, ET AL.: "Phosphor- und Schwefelhydrazine-Verbindungen" ZEITSCHRIFT FÜR NATURFORSCHUNG, vol. 26B, 1971, pages 1232-1235, XP002104845 DIETRICH'SCHE VERLAGSBUCHHANDLUNG, WIESBADEN, DE
- A. RUZICKA, ET AL.: "Zur Synthese von Ammonium-Imide-bis(schwefelsäurefluorid) NH4 N(SO2F)2" ZEITSCHRIFT FÜR CHEMIE, vol. 27, 1987, pages 227-228, XP008121340 DEUTSCHER VERLAG FÜR GRUNDSTOFFINDUSTRIE GMBH, LEIPZIG, DE ISSN: 0044-2402

## Description

### DOMAINE DE L'INVENTION

La présente invention a pour objet des compositions ioniques ayant une conductivité ionique élevée, comprenant un sel dans lequel la charge anionique est délocalisée, et leurs utilisations, notamment comme électrolyte.

### ART ANTÉRIEUR

On connaît depuis longtemps les sels fondus à température ambiante, tel que le nitrate de triéthyle ammonium. Ce composé ne présente pas d'intérêt autre que fondamental du fait de la présence d'un proton labile sur le cation, limitant le domaine de stabilité rédox ou acido-basique de ce composé. On connaît aussi les composés de type méthyl-éthylimidazolium ou butyl-pyridinium, associés à l'ion complexe [Cl⁻, xAlCl3] dans lequel 1< x < 2. Ces composés, du fait de la présence de chlorure d'alurninium sont des acides de Lewis très puissants en plus d'être hygroscopiques et corrosifs car ils libèrent de l'acide chlorhydrique en présence d'humidité. Leur domaine de stabilité électrochimique est limité de plus par l'oxydation anodique des ions chlorure d'une part, et par la réduction des ions aluminium d'autre part.

L'utilisation d'anions réputés stables associés à des cations de type imidazolium ou pyridinium a été proposée, mais les points de fusion sont relativement élevés. Par exemple, l'hexafluorophosphate de 1-méthyl-3-éthylimidazolium fond à 60°C, et l'hexafluorophosphate de 1,2-diméthyl-3-propylimidazolium fond à 65°C. De plus, ces sels, bien que non hygroscopiques, sont néanmoins solubles dans l'eau et peuvent donc difficilement être préparés par échange ionique dans l'eau à moins d'utiliser des substituants alkyles plus longs, ce qui a pour effet de fortement diminuer la conductivité et d'augmenter la viscosité.

US 5,827,602 décrit des sels ayant un point de fusion relativement bas, et dont le critère de choix est un volume de l'anion supérieur à 100 Å³, permettant ainsi d'obtenir des sels à caractère hydrophobe et de conductivité élevée. Les anions les plus représentatifs sont le bis-trifluorométhanesulfonimidure, dont le volume tel que calculé par le programme Hyperchem® est de 144 Å³ ou le tris-trifluorométhanesulfonylméthylure, dont le volume est de 206 Å³.

### SOMMAIRE DE L'INVENTION

La présente invention s'intéresse à une composition électrolytique de bas point de fusion, préférablement inférieur à la température ambiante, dont le cation est de type onium et possède au moins un hétéroatome tel que N, 0, S ou P portant la charge positive et dont l'anion inclut, en totalité ou en partie, au moins un ion imidure du type (FX¹O)N⁻(OX²F) dans lequel X¹ et X² sont identiques ou différents et comprennent SO ou PF, ladite composition contenant au moins un autre composant comprenant un sel métallique, un polymère polaire et/ou un co-solvant aprotique, ladite composition électrolytique étant caractérisée par le fait que ledit composé de bas point de fusion a un cation de type onium de formule : 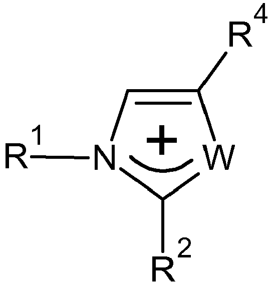 de formule 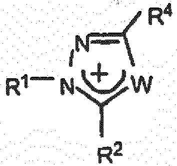 de formule 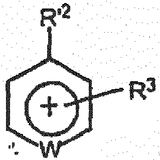 de formule 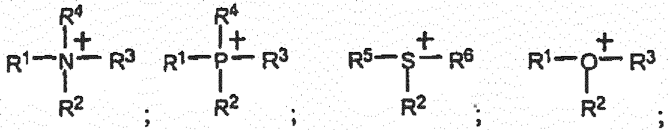 ou 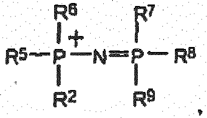 dans lesquelles
W est O, S ou N, et dans lequel N est optionnellement substitué par R¹ lorsque la valence le permet ;
   - R¹, R² et R⁴ sont identiques ou différents et représentent
   - H;
   - les radicaux alkyles, alkényles, oxaalkyles, oxaalkényles, azaalkyles, azaalkényles, thiaalkyles, thiaalkényles, dialkylazo, lesdits radicaux pouvant être linéaires, ramifiés ou cycliques et comprenant de 1 à 18 atomes de carbone;
   - les aryles, arylalkyles, alkylaryles et alkénylaryle de 5 à 26 atomes de carbone comprenant optionnellement un ou plusieurs hétéroatomes dans le noyau aromatique;
   - les groupes comprenant plusieurs noyaux aromatiques ou hétérocycliques, condensés ou non, contenant optionnellement un ou plusieurs atomes d'azote, d'oxygène, de soufre ou de phosphore,
   deux groupements R¹, R³ ou R⁴ pouvant former un cycle ou un hétérocycle de 4 à 9 atomes un ou plusieurs groupements R¹, R³ ou R⁴ sur un même cation peuvent faire partie d'une chaîne polymère;
   - et R² et R⁵ à R⁹ sont identiques ou différents et représentent R¹, R¹O-, (R¹)₂N-, R¹S -, R¹ étant tel que défini précédemment.

### DESCRIPTION DÉTAILLÉE DE L'INVENTION

Il a été trouvé que les sels de cations de type onium variés tels que définis ci-haut, et préférablement les sels imidazolium, ammonium, sulfonium et phosphonium, associés aux anions de la famille représentée par la formule générale (FX¹O)N⁻(OX²F) telle que définie ci-haut permettent d'obtenir des sels liquides à des températures égales ou inférieures à celles obtenues avec des ions plus volumineux. De plus, leur conductivité est, dans tous les cas, à température identique, supérieure à celle des composés décrits dans US 5,827,602. Ces sels liquides sont hydrophobes malgré la faible taille de l'anion, comprise entre 8et 92 Å³ et donc facilement préparés par échange ionique dans l'eau, et peuvent être manipulés sans précaution particulière. De façon inattendue, ces sels ont une stabilité en oxydation égale à celle des anions bis(trifuorométhanesulfonimidure) ou tris(trifuorométhanesulfonyl)méthylure, et supérieure à celle obtenue avec les anions de type tétrafluoroborate ou hexafluorophosphate.

Les compositions électrolytiques de la présente invention peuvent, en plus de l'anion imidure précité, comprendre au moins un autre anion choisi parmi Cl; Br⁻; I; NO₃⁻ M(R¹⁰)₄, A(R¹⁰)₆⁻; R¹¹O₂⁻, [R¹¹ONZ¹], [R¹¹YOCZ²Z³]-, le 4,5-dicyano-1,2,3-triazole, le 3,5bis(R_{f})-1,2,4-triazole, le tricyanométhane, le pentacyanocyclopentadiène, le pentakis(trifluorméthyl)cyclopentadiène, les dérivés de l'acide barbiturique et de l'acide de Meldrum et leurs produits de substitutions ;
- M est B, Al, Ga ou Bi;
- A est P, As et Sb;
- R¹⁰ est un halogène;
- R¹¹ représentant H, F, un groupement alkyle, alkényle, aryle, arylalkyle, alkylaryle, arylalkényle, alkénylaryle, dialkylamino, alcoxy ou thioalcoxy, chacun ayant de 1 à 18 atomes de carbone et étant non substitué ou substitué par un ou plusieurs substituants oxa, thia, ou aza, et dans lesquels un ou plusieurs atomes d'hydrogène sont optionnellement remplacés par un halogène dans une proportion de 0 à 100%, et pouvant éventuellement faire partie d'une chaîne polymère;
- Y représentant C, SO, S=NCN, S=C(CN)₂, POR¹¹, P(NCN)R¹¹, P(C(CN)₂R¹¹, un groupement alkyle, alkényle, aryle, arylalkyle, alkylaryle, arylalkényle, alkénylaryle possédant de 1 à 18 atomes de carbone et optionnellement substitué par un ou plusieurs substituants oxa, thia ou aza; un groupement dialkylamino N(R¹⁰)₂;
- Z¹ à Z³ représentent indépendamment R¹¹, R¹¹YO ou CN, ce groupement pouvant optionnellement faire partie d'une chaîne polymère.

Un autre avantage des compositions électrolytiques de l'invention est le moindre coût des anions de départ, leur préparation ne faisant pas appel à la chimie des perfluoroalkyles tel que CF₃ ou C₄F₉ par exemple, les atomes de fluor présents dans les compositions électrolytiques de l'invention étant dérivés de produit de la chimie inorganique, donc facilement accessibles. Cet aspect économique est particulièrement important car les sels fondus sont constitués de 40 à 75 % en poids de l'espèce anionique, le reste étant l'espèce cationique. De plus, la densité de ces liquides est proche de 1.5 comparé à environ 1 pour les solutions organiques, ce qui requiert des quantités d'autant plus importantes de sels pour toutes les applications où un volume ou une épaisseur donnée sont requises, telles que les films d'électrolytes, réacteurs chimiques etc.

Un autre aspect particulièrement important de la présente invention est la faculté de ces sels fondus de dissoudre d'autres sels, en particulier des sels métalliques, notamment les sels de lithium pour donner des solutions très conductrices. D'une manière similaire les sels fondus, ou leurs mélanges avec d'autres sels métalliques, sont d'excellents solvants ou plastifiants d'un grand nombre de polymères, en particulier ceux portant des fonctions polaires ou ioniques. Aussi bien les composés liquides que les polymères plastifiés par les mélanges ioniques se comportant comme des électrolytes solides sont applicables en électrochimie aux générateurs de type primaire ou secondaires, aux supercapacités, aux systèmes électrochromes, aux revêtements antistatiques, ou encore aux diodes électroluminescentes. La non-volatilité des sels fondus de l'invention, leur stabilité thermique et électrochimiques, et leur conductivité accrue sont des paramètres importants pour la réalisation de systèmes fonctionnant à basse température et ne
présentant pas les risques d'inflammabilité habituels liés à l'emploi de solvants organiques usuels.

Les sels fondus présents dans les compositions électrolytiques de l'invention sont des milieux polaires de faibles volatilité, et par cette propriété, sont capables de servir de solvant permettant d'effectuer un grand nombre de réactions de la chimie organique, telles les substitutions nucléophiles et électrophiles, ou encore les polymérisations anioniques, cationiques ou radicalaires. Il est de plus possible de dissoudre dans ce milieu des catalyseurs, en particulier des sels de métaux de transition ou des terres rares éventuellement cordonnés par des ligands, permettant d'exhalter les propriétés catalytiques. Des exemples de ces catalyseurs incluent les bipyridines, les porphyrines, les phosphines, les arsines. Les organométalliques tels que les métallocènes sont aussi inclus comme solutés pouvant présenter des propriétés catalytiques.

La non-volatilité des sels fondus présents dans les compositions électrolytiques l'invention, leur stabilité thermique ainsi que leur non miscibilité avec les solvants non polaires comme les hydrocarbures, de même que leur caractère hydrophobe, sont particulièrement avantageux pour séparer les produits de réactions chimiques. Il est de même possible de travailler en systèmes diphasiques, le sel fondu contenant le catalyseur et les substrats réactifs étant en solution dans un hydrocarbure ou éther aliphatique non miscible. Après la réaction, une simple décantation permet de séparer la phase organique contenant le produit de la réaction et le sel fondu qui est purifié par lavage avec un non solvant tel que l'eau ou un hydrocarbure, et séché par simple mise sous vide.

Par ailleurs, l'invention a pour objet l'utilisation d'un composé ionique comme milieu pour les réactions chimiques ou électrochimiques mettant en jeu des espèces solubles dans ledit milieu, caractérisée en ce que ledit composé ionique est au moins un composé de bas point de fusion dont le cation est de type onium possédant au moins un hétéroatome tel que N, O, S ou P portant la charge positive et dont l'anion inclut, en totalité ou en partie, au moins un ions imidure du type (FX¹O)N⁻(OX²F) où X¹ et X² sont identiques ou différents et comprennent SO ou PF, ledit composé de bas point de fusion ayant un cation de type onium de formule : 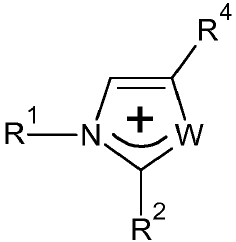 les composés de formule 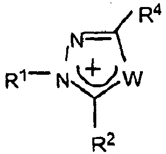 de formule 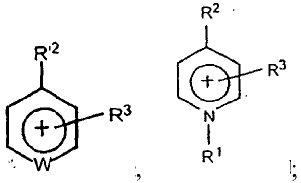 de formule 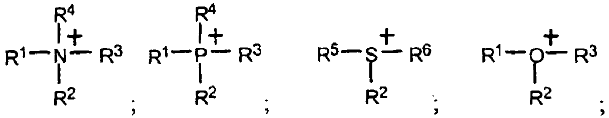 ou 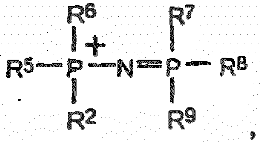 dans lesquelles
- W est O, S ou N, et dans lequel N est optionnellement substitué par R¹ lorsque la valence le permet ;
- R¹, R² et R⁴ sont identiques ou différents et représentent
   - H;
   - les radicaux alkyles, alkényles, oxaalkyles, oxaalkényles, azaalkyles, azaalkényles, thiaalkyles, thiaalkényles, dialkylazo, lesdits radicaux pouvant être linéaires, ramifiés ou cycliques et comprenant de 1 à 18 atomes de carbone;
   - les radicaux cycliques ou hétérocycliques aliphatiques de 4 à 26 atomes de carbone comprenant optionnellement au moins une chaîne latérale comprenant un ou plusieurs hétéroatomes;
   - les aryles, les arylalkyles, alkylaryles et alkénylearyle de 5 à 26 atomes de carbone comprenant optionnellement un ou plusieurs hétéroatomes dans le noyau aromatique ;
   - les groupes comprenant plusieurs noyaux aromatiques ou hétérocycliques, condensés ou non, contenant optionnellement un ou plusieurs atomes d'azote, d'oxygène, de soufre ou de phosphore ;
   deux groupements R¹, R² et R⁴ pouvant former un cycle ou un hétérocycle de 4 à 9 atomes, un ou plusieurs groupements R¹, R² et R⁴ sur un même cation peuvent faire partie d'une chaîne polymère;
   - et R² et R⁵ à R⁹ sont identiques ou différents et représente R¹O-, (R¹)₂N-, R¹S -, R¹ étant tel que défini précédemment.

Les groupements R¹, R³ et R⁴ peuvent porter des groupements actifs en polymérisation tel des doubles liaisons ou des époxydes, ou des fonctions réactives dans les polycondensations, telles que OH, NH₂ et COOH. Dans le cas où les cations portent des doubles liaisons, ils peuvent être homopolymérisés ou copolymérisés, par exemple avec du fluorure de vinylidène, un acrylate, une maléimide, de l'acrylonitrile, un vinyléther, un styrène, etc. Les groupements époxydes peuvent être polycondensés ou copolymérisés avec d'autres époxydes. Ces polycations sont particulièrement utiles seuls ou en mélange avec un solvant, y compris un sel fondu de la présente invention et/ou un ou plusieurs sels de lithium ou un mélange de sels de lithium et potassium comme électrolyte dans les batteries au lithium à anode de lithium ou utilisant une cathode insérant le lithium à bas potentiel comme les spinelles de titane ou des matériaux carbonés.

De préférence, ledit composé ionique est utilisé :
- comme milieu pour les réactions de Diels-Alder, de Friedel-Craft, d'aldolisation, de condensation, de polymérisation anionique, cationique ou radicalaire, et pour les substitutions nucléophiles et électrophiles;
- comme solvant chiral dans une réaction chimique pour la formation d'excès énantiomériques, le cation du composé à bas point de fusion étant un cation ammonium, sulfonium ou phosphonium.

Selon cette utilisation, le milieu contient de préférence au moins une espèce catalytique. L'espèce catalytique est au moins un sel de transition et/ou un sel de terre rare et /ou un sel organométallique tel que les métallocènes, les sels métalliques pouvant être coordonnés avec les ligands.

Les ligands sont choisis dans le groupe constitué par les bipyridines, porphyrines, phosphines, et les arsines.

Le polymère polaire préférentiel comprend des unités monomères dérivées de l'oxyde d'éthylène, l'oxyde de propylène, l'épichlorohydrine, l'épifluorohydrine, le trifluoroépoxypropane, l'acrylonitrile, le méthacrylonitrile, les esters et amides de l'acide acrylique et méthacrylique, le fluorure de vinylidène, la N-méthylpyrrolidone et les polyélectrolytes de type polycation ou polyanion. Finalement, des exemples de co-solvant aprotique préférentiels sont les éthers di-alkyliques de l'éthylène glycol, du diéthylène glycol, du tri éthylène glycol, des polyéthylène glycols de masse comprise entre 400 et 2000; les esters, en particulier ceux de l'acide carbonique, linéaires ou cyclique tels le diméthylcarbonate, le méthyl-éthylcarbonate, le diéthylcarbonate, le carbonate d'éthylène, le carbonate de propylène; les esters comme la γ-butyrolactone, les nitriles comme le glutaronitrile, le 1,2,6-tricyanohexane, les amides comme le diméthylformamide, la N-méthylpyrrolidinone, les sulfamides et sulfonamides ainsi que les mélanges des composés précités.

Lorsque la présente composition électrolytique comprend plus d'un polymère, au moins un de ces derniers peut être réticulé.

L'invention a également pour objet un générateur électrochimique ayant au moins une électrode positive et au moins une électrode négative, caractérisé en ce qu'il utilise une composition électrolytique de l'invention.

Un générateur électrochimique comprenant une composition électrolytique de l'invention contient préférentiellement une électrode négative contenant soit du lithium métallique ou un de ses alliages, soit un composé d'insertion du carbone, en particulier du coke de pétrole ou du graphite, soit un oxyde à bas potentiel d'insertion tel que les spinelles de titane Li_{4-x+3y}T₅₋ₓO₁₂ (0≤x, y≤1), soit un nitrure double d'un métal de transition et de lithium comme Li₃₋ₓCo_{z}N (0≤z≤1) ou ayant la structure de type antifluorite comme Li₃FeN₂ ou Li₇MnN₄, ou leurs mélanges. L'électrode positive du générateur contient préférentiellement soit de l'oxyde de vanadium VOₓ (2 ≤ x ≤ 2,5), soit de l'oxyde mixte de lithium et de vanadium LiV₃0₈, soit un oxyde double de cobalt et de lithium optionnellement partiellement substitué de formule générale Li_{1-α}Co₁-_{x+y}NiₓAl_{y} (0≤x+x≤1 ; 0≤y≤0,3 ; 0≤α≤1), soit un spinelle de manganèse optionnellement partiellement substitué de formule générale Li_{1-α}Mn_{2-z}M_{z} (0≤z≤1) où M = Li, Mg, Al, Cr, Ni, Co, Cu, Ni, Fe, soit un phosphate double de structure olivine ou Nasicon tels que Li_{1-α}Fe₁₋ₓMnₓPO₄,Li_{1-x+2α}Fe₂P₁₋ₓSixO₄ (0≤x, α≤1), soit un sel de l'acide rhodizonique, soit un polydisulfure dérivé de l'oxydation du dimercaptoéthane, du 2,5dimercapto-1,3,4-thiadiazole, 2,5-dimercapto-1,3,4-oxadiazole, du 1,2-dimercaptocyclobutène-3,4-dione, ou leurs mélanges.

De façon avantageuse, au moins une des électrodes du générateur est mélangée à la composition électrolytique pour former une électrode composite.

La composition électrolytique de l'invention peut en outre être utilisée comme électrolyte dans un système de stockage de l'énergie électrique de type supercapacité, contenant optionnellement dans une électrode du carbone de grande surface spécifique, ou un polymère conjugué. L'invention a donc pour objet un système de stockage de l'énergie électrique de type supercapacité, caractérisé en ce qu'il utilise une composition électrolytique de l'invention. De façon avantageuse, le polymère conjugué contient 3 degrés d'oxydation, et se retrouve dans les 2 électrodes. Un exemple d'un tel polymère est un dérivé du phényl-3-thiophène.

Finalement, la composition électrolytique de l'invention peut être utilisée comme électrolyte dans un système de modulation de la lumière de type électrochrome comprenant au moins un matériau électrochrome. L'invention a donc également pour objet un système de modulation de la lumière de type électrochrome comprenant au moins un matériau électrochrome, caractérisé en ce qu'il utilise une composition électrolytique de l'invention. Dans un tel système, le matériau électrochrome est avantageusement déposé sur une couche d'un semi-conducteur transparent dans le visible, préférentiellement un dérivé de l'oxyde d'étain ou de l'oxyde d'indium, sur un substrat de verre ou d'un polymère. Des exemples de matériaux électrochromes préférentiels incluent l'oxyde de molybdène, de tungstène, de titane, de vanadium, de niobium, de cérium, d'étain, ainsi que leurs mélanges. Le matériau électrochrome peut être optionnellement dissous dans l'électrolyte.

Les exemples suivants sont fournis afin d'illustrer des mises en oeuvre préférentielles de l'invention, et ne doivent pas être considérés comme en limitant sa portée.

### Exemple 1

15 g de chlorure de 1-méthyl-3-éthyl imidazolium EMICI (C₆H₁₁N₂Cl) sont dissous dans 100 ml d'eau auquel sont ajoutés sous agitation 23 g de bis-fluorosulfonimidure de potassium (KFSI) K[(FSO₂)₂N]. Une séparation en deux phases liquide se produit immédiatement. Le sel fondu bis-fluorosulfonimidure de 1-méthyl-3-éthyl imidazolium (EMIFSI) est extrait par le dichlorométhane et séché par du sulfate de magnésium anhydre. La suspension est filtrée et le solvant évaporé. Le sel est séché sous vide à 80°C correspond à la formule développée:

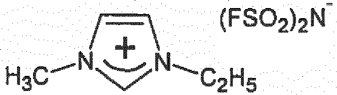

Ce composé ionique examiné par DSC présente un point de fusion de -15°C. La perte de poids mesurée par analyse thermique différentielle (ATD) sous argon est inférieure à 1% jusqu'à 350°C. La conductivité en fonction de la température est donnée dans le Tableau 1 ci-dessous :

**TABLEAU 1**

| Température (°C) | -10 | 0 | 10 | 20 | 30 | 40 | 50 | 60 |
|---|---|---|---|---|---|---|---|---|
| Conductivité (mScm⁻¹) | 5.6 | 8.3 | 11 | 15 | 20 | 25 | 31 | 37 |
| σ (FSI) / σ TFSI (%) | 2.41 | 2.21 | 2.01 | 1.86 | 1.80 | 1.70 | 1.67 | 1.64 |

La conductivité est supérieure à celle obtenue avec le sel bis-trifluorométhanesulfonimidure de 1-méthyl-3-éthyl imidazolium (EMITFSI). Le rapport entre les valeurs de conductivité σ entre le sel de l'invention, i.e, fluorosulfonimidure de 1-méthyl-3-éthyl imidazolium (noté FSI dans le tableau) et bis-trifluorométhanesulfonimidure de 1-méthyl-3-éthyl imidazolium (noté TFSI dans le tableau) est donné dans la dernière ligne du tableau précédent. Ces chiffes montrent la très nette amélioration des performances de conductivité par rapport à l'art antérieur.

Le domaine de stabilité électrochimique mesurée par voltammétrie cyclique sur électrode de nickel pour les potentiels cathodiques et de carbone vitreux pour les potentiels anodiques est de 5.2 Volts (0 ⇒ 5.2 V vs. Li⁺ /Li⁰).

### Exemple 2 (ne faisant pas partie de l'invention revendiquée)

Le bis-difluorophosphonylamidure de lithium Li[(POF₂)₂N] est préparé selon la méthode de Fluck et Beuerle dans Z. Anorg. Allg. Chem., 1975, 412, 65, par réaction du dérivé lithié de l'hexaméthyldisilazane sur l'oxyfluorure de phosphore selon la réaction suivante:

2 POF₃ + Li[Si(CH₃)₃]N ⇒ 2 FSi(CH₃)₃ + Li[(POF₂)₂N]

Le sel ionique fondu bis-difluorophosphonylimidure de 1-méthyl-3-éthyl imidazolium est préparé par échange ionique dans l'eau de selon l'exemple 1 entre 10 g de EMICI et 13 g Li[(POF₂)₂N] et extraction au dichlorométhane. Le sel fondu a des propriétés physico-chimiques similaires à celles du sel de fluorosulfonyle de l'exemple 1.

### Exemple 3

Différents sels d'imidazolium de formule générale : 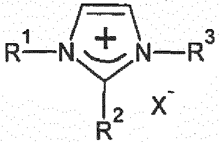 ont été préparés avec les anions [(FSO₂)₂N]⁻ et [(POF₂)₂N]⁻, et sont illustrés dans le Tableau 2 ci-dessous. Ceux avec un indice "+" sont des sels liquides à température ambiante.

**TABLEAU 2**

| R³ | CH₃ | C₂H₅ | C₃H₇ | C₄H₉ | C₅H₁₁ | C₇H₁₅ | C₈H₁₇ |
|---|---|---|---|---|---|---|---|
| R¹ = CH₃, R² = H | | + | + | + | + | + | + |
| R¹ = CH₃, R² = CH₃ | | | + | + | + | + | + |
| R¹ = C₂H₅, R² = H | + | + | + | + | + | + | + |
| R¹ = CH₃, R² = C₂H₃ | + | + | + | + | + | + | + |

### Exemple 4 (ne faisant pas partie de l'invention revendiquée)

10 g de bromure de triéthylhéxyl ammonium commercial (C₁₂H₂₈NBr) sont dissous dans 150 ml d'eau auxquels sont ajoutés sous agitation 8.5 g de bisfluorosulfonimidure de potassium [K(FSO₂)₂N]. Le sel fondu bis-fluorosulfonimidure de triéthylhéxyl ammonium est séparé par centrifugation et lavé par trois aliquotes de 50 ml d'eau puis extrait par 30 ml de dichlorométhane et séché par du sulfate de magnésium anhydre. La suspension est filtrée et le solvant évaporé, laissant un liquide visqueux. La conductivité à 25°C est supérieure à 5 x 10⁻⁴ Scm⁻¹ à 25°C.

### Exemple 5

10 g de diméthyléthylamine commerciale et 11 ml de bromo-1-propane sont mis au reflux dans 40 ml d'acétonitrile pendant 48 heures. Le solvant est ensuite évaporé et le résidu solide est lavé à l'éther. À 12 g du sel (CH₃)₂(C₂H₅)(C₃H₇)NBr dissous dans 75 ml d'eau sont ajoutés 13 g de bis-fluorosulfonimidure de potassium [K(FSO₂)₂N]. Le sel fondu est extrait comme précédemment et se présente sous forme d'un liquide peu visqueux. Sa conductivité en fonction de différentes températures est donnée dans le Tableau 3 suivant.

**Tableau 3**

| Température (°C) | | 20 | 30 | 40 | 50 |
|---|---|---|---|---|---|
| conductivité (mScm⁻¹) | FSI | 4,95 | 7,02 | 9,4 | 12,3 |
| | TFSI | 1,81 | 2,92 | 4,3 | 6,1 |

A titre de comparaison, la valeur de la conductivité du sel bis-trifluorométhanesulfonimidure de diméthyléthylpropyl-ammonium est donnée (Tableau 3, ligne 3). La conductivité du composé selon l'invention apparaît de 2,5 à 2 fois plus élevée que celle du sel équivalent faisant intervenir un anion plus volumineux.

### Exemple 6

La N-méthyl-N-éthyl-aniline est quaternarisée par le bromopropane au reflux dans l'acétonitrile pendant 48 heures. Le sel est obtenu par évaporation du solvant et purifié par lavage du résidu solide à l'éther. 5 g du sel obtenu sont dissous dans 25 ml d'eau, et 4.6 g de bis-fluorosulfonimidure de potassium (K(FSO₂)₂N) y sont ajoutés. Le sel fondu bis-fluorosulfonimidure de méthyléthylpropylphényl ammonium est extrait par 15 ml de dichlorométhane, lavé par trois aliquotes de 50 ml d'eau et séché par du sulfate de magnésium anhydre. Ce sel existe sous deux isomères optiques qui peuvent être séparés sur une colonne chirale ou par précipitation du sel de camphre-sulfonate à partir du bromure avant échange par l'imidure. Ce sel peut servir de milieu réactionnel chiral.

### Exemple 7

Le bromure de N-butylpyridinium est préparé par action du bromobutane sur la pyridine en l'absence de solvant à 45°C. À 5 g de sel dissous dans 35 ml d'eau sont ajoutés 4.6 g de bis-diflurophosphonylimidure de lithium Li[(POF₂)₂N]. Le sel liquide est traité d'une manière similaire à celle des sels fondus obtenus dans les exemples précédents et est finalement séché sous vide à 60°C. Le sel fondu bis-fluorosulfonimidure de N-propyl pyridinium est préparé d'une manière similaire à partir du sel de potassium correspondant.

### Exemple 8

Le sulfure d'éthylméthyle commercial (Aldrich, Milwaukee USA) est quaternarisé par le sulfate de propyle (TCI, Japon). Le propylsulfate de diéthylméthylpropylsulfonium est traité en solution aqueuse par 1 équivalent de bis-fluorosulfonimidure de potassium. Le sel fondu liquide est extrait comme précédemment. D'une manière similaire à celle employée pour l'exemple 4, ce sel peut être dédoublé en deux isomères optiquement actifs et servir à induire un excès énantiomérique pour les réactions effectuées lorsque le sel est utilisé comme solvant.

### Exemple 9

15 g de chlorhydrate de 4-chloropyridine commercial sont dissous dans 100 ml d'eau auxquels sont ajoutés 8.5 g de bicarbonate de sodium. La 4-chloropyridine est extraite par l'éther et séchée par le sulfate de magnésium, et le solvant est évaporé. 10 g de 4-chloropyridine dans 60 ml d'acétonitrile sont quaternarisés par 15.6 g de trifluorométhanesulfonate d'éthyle et sont ajoutés 11.6 g de triméthylsilyléthylméthyl-amine C₂H₅(CH₃)NSi(CH₃)₃. Le mélange réactionnel est mis au reflux pendant une heure puis refroidi. Le solvant est évaporé et le résidu solide est repris par l'eau. À cette solution sont ajoutés 19.5 g de bis-fluorosulfonimidure de potassium. Le sel liquide qui se décante est extrait au dichlorométhane. Ce sel a pour structure:

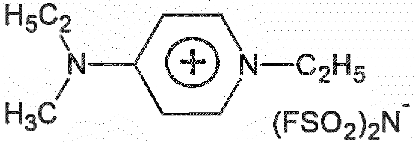

### Exemple 10

La fluorosulfonamide FSO₂NH₂ est préparée par action de l'anhydride fluorosulfonique (15 g) sur la carbamate d'ammonium (12 g) en suspension dans le dichlorométhane selon la réaction suivante :

(FSO₂)₂O + 1,5 H₂NCO₂(NH₄) ⇒ FSO₃(NH₄) + 1,5 CO₂ + FSO₂NH(NH₄)

Le mélange réactionnel est filtré. L'amide FSO₂NH₂ est libérée par l'acide chlohrydrique dilué et extrait à l'éther. Le dérivé triméthylsilylé du dérivé sodique de la fluorosulfonamide est préparé par la méthode de Foropoulous et al. dans Inorganic Chem., 1984, 23, 37. Dans un réacteur Parr® sont mis 80 ml d'acétonitrile anhydre et 10 g du dérivé de la fluorosulfonamide. Le réacteur est fermé et purgé sous azote et sont admis 5.38 g de fluorure de phosphoryle POF₃ en maintenant la température à 45°C. La pression tombe après une heure et le réacteur est refroidi et ouvert. Le sel de sodium de l'imide mixte a été obtenu selon la réaction

NaNSi(CH₃)₃SO₂F + POF₃ ⇒ Si(CH₃)₃ + Na[(F₂PO)(FSO₂)N]

Le sel est recueilli par évaporation du solvant et recristallisé dans un mélange toluène-acétonitrile. Ce sel donne des dérivés ioniques liquides avec les imidazolium de l'exemple 3 et présente un domaine de liquides plus large que celui des sels de bis-trifluorométhanesulfonimidure et une conductivité supérieure de 10 à 25 %.

### Exemple 11

25 ml d'une solution commerciale de chlorure de diallyldiméthyl-ammonium à 65% dans l'eau sont dilués par 100 ml d'eau et sont ajoutés sous agitation 22 g de bis-fluorosulfonimidure de potassium. Le précipité liquide est extrait par le dichlorométhane et séché par le sulfate de magnésium. Ce sel fondu a pour formule développée :

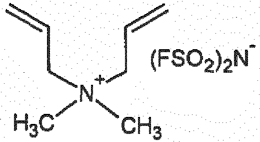

Il se comporte comme un monomère actif en polymérisation radicalaire pour former par cyclopolymérisation des motifs bis(3,5-méthylène-) diméthylpyrrolidinium. Ce composé donne, outre des homopolymères, des copolymères avec le styrène, l'anhydride maléique, les N-maléimides, le fluorure de vinylidène, l'acrylonitrile, le méthacrylonitrile, le méthacrylate de méthyle, avec les acrylates ou méthacrylates de ω̅-methoxyoligo ethylène glycols de masse comprise entre 200 et 2000 daltons, éventuellement réticulés par un diacrylate ou méthacrylate de a, ω̅-oligo ethylène glycol.

### Exemple 12

5 g de pentachlorure de phosphore sont dissous dans 50 ml de dichlorométhane dans un ballon muni d'une ampoule à brome et d'une entrée d'argon sec. Le mélange est refroidi par de la glace carbonique à -78°C et on ajoute goutte à goutte 20 ml de méthyléthylamine dans 30 ml d'acétonitrile anhydre par l'ampoule à brome. Le mélange réactionnel est maintenu sous agitation pendant 1 heure en le laissant revenir à température ordinaire. Le solvant est ensuite évaporé et le résidu est repris dans 75 ml d'eau et filtré sur Celite®. À cette solution sont ajoutés 5.5 de bis-fluorosulfonimidure de potassium. Le milieu réactionnel se sépare en deux phases liquides. Le sel fondu de tetrakis(éthylméthylamino)phosphonium {P[N(CH₃)(C₂H₅)]}⁺[(FSO₂)₂N]⁻ est un liquide huileux à température ordinaire. Ce sel fondu est particulièrement stable vis-à-vis des agents réducteurs ou nucléophiles, et ce, même à des températures élevées.

### Exemple 13 (exemple ne faisant pas partie de l'invention revendiquée)

20 g d'une solution aqueuse commerciale à 25 % de poly(chlorure de diallyldiméthylammonium) de haute masse moléculaire (M_{w} environ 2 x 10⁵) sont dilués dans 100 ml d'eau. Sous agitation magnétique sont ajoutés 6.7 g de bis-fluorosulfonimidure de potassium dans 100 ml d'eau. Le précipité de poly(bis-fluorosulfonimidure de diallyldiméthyl-ammonium) 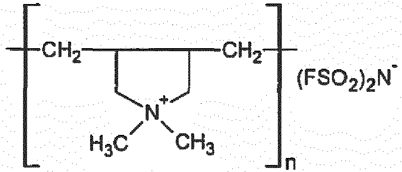 est ensuite filtré et lavé abondamment à l'eau distillée, puis séché sous vide.

### Exemple 14 (exemple ne faisant pas partie de l'invention revendiquée)

Un électrolyte liquide est obtenu par dissolution du bis-trifluorométhanesulfonylimidure de lithium (LiTFSI) en concentration 1 molaire dans le sel fondu préparé selon l'exemple 1. La conductivité de ce mélange est de 9 x 10⁻³ Scm⁻¹ à 25°C, et reste supérieure à 2 x 10⁻³ Scm⁻¹ à 0°C. Par voltammétrie cyclique, le domaine anodique est trouvé supérieur à V/Li°/Li⁺.

### Exemple 15 (exemple ne faisant pas partie de l'invention revendiquée)

Un électrolyte solide est obtenu par plastification du polyélectrolyte de l'exemple 13 par la solution de sel de lithium dans le sel d'imidazolium de l'exemple 14. Pour la mise en forme de cet électrolyte, les 3 composants (polyélectrolyte-FSI, imidazolium-FSI, LiTFSI sont pesés de manière à respecter les proportions suivantes: polyélectrolyte (40 % en poids; LiTFSI, 1 M dans le sel d'imidazolium (60 % en poids). Les trois composants sont dissous dans un solvant polaire volatile, tel que l'acétonitrile, la quantité de solvant étant ajustée de manière à permettre l'épandage en film mince de la solution pour donner après séchage une épaisseur de 35 µm sur un support de polypropylène.

Le film ainsi obtenu est séché par un courant d'air sec, puis sous vide primaire à 100°C pendant deux heures. Toutes les manipulations successives de ce film sont faites en boîte à gants (< 1 ppm O₂ et H₂O). La conductivité de cet électrolyte est égale à 10⁻³ Scm⁻¹ à 20°C ; 4 x 10⁻⁴ Scm⁻¹ à 0°C; et 3x10⁻³ Scm⁻¹ à 60°C. Des électrolytes de conductivité supérieure peuvent être obtenus en augmentant la fraction de plastifiant (> 60%), i.e., la solution de LiTFSI dans le sel fondu de l'exemple 1. D'une manière similaire, des modules d'élasticité supérieurs sont obtenus pour des fractions de plastifiant inférieures à <50% avec une diminution de la conductivité.

### Exemple 16 (exemple ne faisant pas partie de l'invention revendiquée)

Un électrolyte polymère de conductivité élevée est obtenu par plastification à raison de 40% en poids par le composé ionique de l'exemple 2 d'un complexe polyéther-sel métallique. Le complexe est à base de bis-trifluorométhanesulfonimidure de lithium et de poly(oxyde d'éthylène) de masse moléculaire 5 x 10⁶, tel que le rapport des oxygènes du polymère au nombre d'ions lithium soit égal à 20 (O : Li = 20:1).
L'électrolyte peut être préparé directement par co-dissolution des composants pesés selon les proportions stoechiométriques dans un solvant tel que l'acétonitrile et évaporation suivie d'un séchage sous vide à 80°C.

Dans une variante, l'homopolymère d'oxyde d'éthylène peut être remplacé par un copolymère d'oxyde d'éthylène et d'allylglycidyléther (5% molaire) auquel sont ajoutés 1 % en poids d'Irgacure 651 ®. La solution dans l'acétonitrile est épandue sur un support de polypropylène à l'aide d'un gabarit pour former un film de 20 microns d'épaisseur après séchage. Sous balayage d'argon, le film est soumis au rayonnement UV produit par une lampe de type Hanovia® ayant son maximum d'émission à 254 nm. L'éclairement correspond à une énergie de 130 m Wcm⁻². Le polymère se réticule par un processus radicalaire par les segments insaturés et présente alors d'excellentes propriétés mécaniques de type élastomère. Des mélanges ternaires sel fondu / sel de lithium / polymère peuvent être obtenus d'une manière similaire avec comme matériau macromoléculaire soit l'acrylonitrile; soit le fluorure de polyvinylidène et ses copolymères avec l'hexafluoropropène, en particulier ceux solubles dans l'acétone et permettant une mise en oeuvre aisée; ou encore soit le polyméthacrylate de méthyle.

### Exemple 17 (exemple ne faisant pas partie de l'invention revendiquée)

Un électrolyte polymère est préparé par polymérisation *in situ* d'un mélange du monomère de l'exemple 11 (25% en poids), du bis-trifluorométhanesulfonimidure de lithium (24%) et du sel fondu de l'exemple 1 (45%), et 1 % de l'amorceur radicalaire 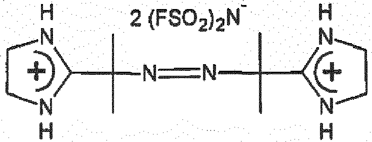 Cet amorceur est obtenu par échange dans l'eau à partir du chlorure commercial (Wako, Japon) et de K(FSO₂)₂N). Le mélange liquide est épandu à l'aide d'un gabarit sous forme de film de 30 microns d'épaisseur sur un support de polypropylène, et polymérisé dans un four tunnel sous atmosphère d'azote à 80°C pendant 1 heure. L'électrolyte ainsi obtenu est un élastomère possédant un domaine de stabilité anodique supérieur à 5V et une conductivité supérieure à 3 x 10⁻⁴ Scm⁻¹ à 25°C.

### Exemple 18 (exemple ne faisant pas partie de l'invention revendiquée)

Un générateur électrochimique secondaire est fabriqué comprenant comme matériau actif de l'électrode positive de l'oxyde double cobalt et de lithium, LiCoO₂, et comme matériau actif de l'électrode négative le spinelle de titane et de lithium Li₄Ti₅O₁₂. L'électrolyte est préparé selon l'exemple 14 sous forme de film de 25 microns. Chaque électrode du type composite est préparée par épandage d'une suspension du matériau actif, de noir de carbone (Ketjenblack®) dans une solution dans le cyclohexane d'un copolymère d'éthylène-copropylène-diène (Aldrich, USA). La composition finale correspond à 90% en volume du matériau actif, 5% v/v de noir de carbone et 5% de copolymère. Après épandage sur collecteurs de courant en aluminium de 8 microns d'épaisseur, l'électrode négative contient 16.4 mg de matériau actif par cm² soit 2.9 mAhcm⁻², et l'électrode positive 16,5 mg de matériau actif par cm², soit 2.7 mAhcm⁻². Les électrodes et leur collecteur de courant sont découpés en carrés de 4 cm² et sont placées de part et d'autre d'une membrane de polyéthylène microporeux (Celgard®) imbibée de l'électrolyte liquide préparé selon l'exemple 14. La batterie ainsi assemblée est caractérisée par voltammétrie lente à l'aide d'un appareil de type MacPile® (Claix France). 92% de la capacité de l'électrode positive sont obtenus dans le domaine de voltage 2 - 2.8 V à une vitesse de balayage de 10 mV.mn⁻¹. La densité d'énergie dans cette configuration est de 8Wh.kg⁻¹.

### Exemple 19 (exemple ne faisant pas partie de l'invention revendiquée)

Un générateur électrochimique secondaire est fabriqué comprenant comme matériau actif de l'électrode positive le phosphate double de manganèse dopé au fer et de lithium, LiMn_{0,9}Fe_{0,1}PO₄, et comme matériau actif de l'électrode négative le spinelle de titane et de lithium Li₄Ti₅O₁₂. L'électrolyte est préparé selon l'exemple 15 sous forme de film de 25 microns. Chaque électrode du type composite est préparée par épandage d'une suspension de 45% en volume du matériau actif, 5% v/v de noir de carbone (Ketjenblack®) et d'une solution dans l'acétonitrile des composants de l'électrolyte selon l'exemple 12 (50% v/v). Les collecteurs de courant sont en aluminium de 8 microns d'épaisseur. Le collecteur de l'électrode positive est recouvert d'un enduit protecteur de graphite (Acheson, USA). Après épandage, l'électrode négative a une charge de 12 mg de matériau actif par cm² soit 2.2 mAhcm⁻², et l'électrode positive 14 mg de matériau actif par cm² soit 2.4 mAhcm⁻². Les électrodes et leur collecteurs de courant sont découpés en carrés de 4 cm² et sont placées de part et d'autre de l'électrolyte et l'assemblage est laminé à 80°C pour assurer un bon contact aux interfaces. La batterie ainsi assemblée est caractérisée par voltammétrie lente. 82% de la capacité de l'électrode positive sont obtenus dans le domaine de voltage 2.6 - 3.2 V à une vitesse de balayage de 10 mV.mn⁻¹. La densité d'énergie dans cette configuration est proche de 100 Wh.kg⁻¹.

### Exemple 20

Une supercapacité est élaborée à partir d'électrodes de fibres de carbone activé de 900 m²g⁻¹ (Spectracarb®), Deux carrés de 4 cm² sont découpés dans le tissu de carbone et sont imbibées sous vide par l'électrolyte préparé selon l'exemple 1. Les deux électrodes symétriques sont séparées par une membrane de polyéthylène poreux (Celgard®) imbibée sous vide du même liquide ionique. Les deux collecteurs de courant sont en aluminium de 10 µm recouvert par pulvérisation cathodique d'une couche protectrice de 5000 Å de molybdène. La capacité volumique pour une tension maximale de charge de 2.8 V est de 12 Fcm⁻³, soit 3 Wh.L⁻¹ au seuil de coupure de tension de 1.2 V.

### Exemple 21

Le sel d'imidazolium de l'exemple 1 est utilisé comme solvant du bis-trifluorométhanesulfonimidure d'yttrium en concentration de 0.1M. Ce liquide est utilisé comme catalyseur de la réaction de Diels-Alder du cyclopentadiène sur l'acrylate de méthyle. Les réactifs sont mélangés en quantité stoechiométrique, et le liquide ionique est ajouté à raison de 30% v/v. Sous agitation, la réaction est complétée en 1 heure à 25°C. Les produits de la réaction sont extraits avec de l'hexane, qui est non miscible avec le composé ionique. Le rapport endo/exo est de 9:1. Le catalyseur traité à 100°C sous vide peut être réutilisé sans perte de son activité.

### Exemple 22

Le sel fondu préparé selon l'exemple 12 est utilisé comme solvant pour des réactions de substitution nucléophile. 10 g de ce sel et 3 g de cyanure de potassium sont mis dans un tube de verre dans un four, et la température est portée à 60°C. 4 g de chlorure de benzyle sont chauffés à 60°C pendant 2 heures. Le rendement de conversion du chlorure de benzyle en cyanure de benzyle est de 85%. Le sel fondu peut être aisément recyclé par lessivage à l'eau et évaporation.

## Revendications

1. Composition électrolytique comprenant au moins un composé de bas point de fusion dont le cation est de type onium possédant au moins un hétéroatome tel que N, O, S ou P portant la charge positive et dont l'anion inclut, en totalité ou en partie, au moins un ion imidure du type (FX¹O)N⁻(OX²F) où X¹ et X² sont identiques ou différents et comprennent SO ou PF, ladite composition contenant au moins un autre composant comprenant un sel métallique, un polymère polaire et/ou un co-solvant aprotique, ladite composition électrolytique étant **caractérisée par le fait que** ledit composé de bas point de fusion a un cation de type onium de formule : 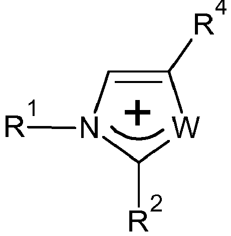 de formule 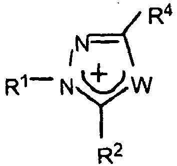 de formule 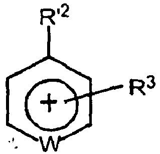 de formule 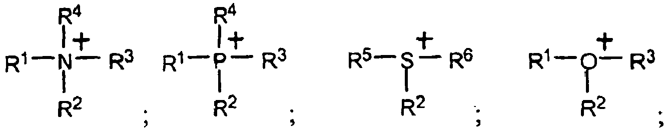 ou 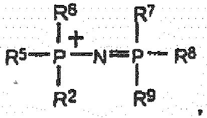 dans lesquelles
- W est O, S ou N, et dans lequel N est optionnellement substitué par R¹ lorsque la valence le permet ;
- R¹, R³ et R⁴ sont identiques ou différents et représentent
- H;
- les radicaux alkyles, alkényles, oxaalkyles, oxaalkényles, azaalkyles, azaalkényles, thiaalkyles, thiaalkényles, dialkylazo, lesdits radicaux pouvant être linéaires, ramifiés ou cycliques et comprenant de 1 à 18 atomes de carbone;
- les radicaux cycliques ou hétérocycliques aliphatiques de 4 à 26 atomes de carbone comprenant optionnellement au moins une chaîne latérale comprenant un ou plusieurs hétéroatomes;
- les aryles, arylalkyles, alkylaryles et alkénylaryle de 5 à 26 atomes de carbone comprenant optionnellement un ou pusiuers hétéroatomes dans le noyau aromatique ;
- les groupes comprenant plusieurs noyaux aromatiques ou hétérocycliques, condensés ou non, contenant optionnellement un ou plusieurs atomes d'azote, d'oxygène, de soufre ou de phosphore,
deux groupements R¹, R³ ou R⁴ pouvant former un cycle ou un hétérocycle de 4 à 9 atomes un ou plusieurs groupements R¹, R³ ou R⁴ sur un même cation peuvent faire partie d'une chaîne polymère;
- et R² et R⁵ à R⁹ sont identiques ou différents et représentent R¹, R¹O-, (R¹)₂N-, R¹S-, R¹ étant tel que défini précédemment

2. Composition électrolytique selon la revendication 1, dans laquelle les groupements R¹, R³ et R⁴ peuvent porter un groupement actif en polymérisation.

3. Composition électrolytique selon la revendication 2, dans laquelle le groupement actif en polymérisation comprend les doubles liaisons, les époxydes, ou les fonctions réactives dans les polycondensations.

4. Composition électrolytique selon la revendication 1, dans laquelle le cation comprend un ion ammonium, imidazolium, pyridinium ou phosphonium non substitué ou substitué par un groupement alkyle, oxaalkyle ou dialkylamino comprenant de 1 à 8 atomes de carbone.

5. Composition électrolytique selon l'une quelconque des revendications 1 à 4, qui contient en outre au moins un anion choisi choisi parmi Cl; Br-; I-, NO₃⁻ M(R¹⁰)₄⁻, A(R¹⁰)₆⁻; R¹¹O₂⁻, [R¹¹ONZ¹]⁻, [R¹¹YOCZ²Z³]⁻, le 4,5-dicyano-1,2,3-triazole, le 3,5bis(Rf)-1,2,4-triazole, le tricyanométhane, le pentacyanocyclopentadiène, le pentakis(trifluoro-méthyl)ocycloopentadiène, les dérivés de l'acide barbiturique et de l'acide de Meldrum et leurs produits de substitutions ;
- M est B, Al, Ga ou Bi;
- A est P, As et Sb;
- R¹⁰ est un halogène;
- R¹¹ représentant H, F, un groupement alkyle, alkényle, aryle, arylalkyle, alkylaryle, arylalkényle, alkénylaryle, dialkylamino, alcoxy ou thioalcoxy, chacun ayant de 1 à 18 atomes de carbone et étant non substitué ou substitué par un ou plusieurs substituants oxa, thia, ou aza, et dans lesquels un ou plusieurs atomes d'hydrogène sont optionnellement remplacés par un halogène dans une proportion de 0 à 100%, et pouvant éventuellement faire partie d'une chaîne polymère;
- Y représentant C, SO, S=NCN, S=C(CN)₂, POR¹¹, P(NCN)R¹¹, P(C(CN)₂R¹¹, un groupement alkyle, alkényle, aryle, arylalkyle, alkylaryle, arylalkényle, alkénylaryle possédant de 1 à 18 atomes de carbone et optionnellement substitué par un ou plusieurs substituants oxa, thia ou aza; un groupement dialkylamino N(R¹⁰)₂;
- Z¹ à Z³ représentent indépendamment R¹¹, R¹¹YO ou CN, ce groupement pouvant optionnellement faire partie d'une chaîne polymère.

6. Composition électrolytique selon la revendication 1, **caractérisé en ce que** le cation du sel métallique est un cation d'un métal alcalin, d'un métal alcalino-terreux, d'un métal de transition et d'une terre rare.

7. Composition électrolytique selon la revendication 6, **caractérisé en ce qu'**au moins un sel métallique est un sel de lithium.

8. Composition électrolytique selon la revendication 1, **caractérisé en ce que** le polymère polaire comprend des unités monomères dérivées de l'oxyde d'éthylène, l'oxyde de propylène, l'épichlorohydrine, l'épifluorohydrine, le trifluoroépoxypropane, l'acrylonitrile, le méthacrylonitrile, les esters et amides de l'acide acrylique et méthacrylique, le fluorure de vinylidène, la N-méthylpyrolidone et les polyélectrolytes de type polycation ou polyanion.

9. Composition électrolytique selon la revendication 8, **caractérisé en ce que** au moins un des polymères est réticulé lorsque la composition comprend plus d'un polymère.

10. Composition électrolytique selon la revendication 1, **caractérisé en ce que** le co-solvant aprotique est choisi parmi les éthers di-alkyliques de l'éthylène glycol, du diéthylène glycol, du triéthylène glycol, des polyéthylène glycols de masse comprise entre 400 et 2000; les esters, en particulier ceux de l'acide carbonique, linéaires ou cyclique tels le diméthylcarbonate, le méthyl-éthylcarbonate, le diéthylcarbonate, le carbonate d'éthylène, le carbonate de propylène ; les esters comme la -butyrolactone, les nitriles comme le glutaronitrilc, le 1,2,6-tricyanohexane, les amides comme le diméthylformamide, la N-méthylpyrrolidinone, les sulfamides et sulfonamides ainsi que les mélanges des composés précités.

11. Générateur électrochimique ayant au moins une électrode positive et au moins une électrode negative, **caractérisé en ce qu'**il utilise une composition électrolytique selon l'une quelconque des revendications 1-10.

12. Générateur électrochimique selon la revendication 11, **caractérisé en ce que** ladite composition électrolytique contient au moins un sel de lithium.

13. Générateur électrochimique selon la revendication 11 ou 12, **caractérisé en ce que** l'électrode négative contient soit du lithium métallique ou un de ses alliages, soit un composé d'insertion du carbone, en particulier du coke de pétrole ou du graphite, soit un oxyde à bas potentiel d'insertion tel que les spinelles de titane Li_{4-x+3y}Ti₅₋ₓO₁₂ (0≤x, y≤1), soit un nitrure double d'un métal de transition et de lithium comme Li₃₋ₓCo_{z}N (0≤z≤1) ou ayant la structure de type antifluorite comme Li₃FeN₂ ou Li₇MnN₄, ou leurs mélanges.

14. Générateur électrochimique selon la revendication 11 ou 12, **caractérisé en ce que** l'électrode positive contient soit de l'oxyde de de l'oxyde de vanadium VOx (2≤ x ≤ 2,5), soit de l'oxyde mixte de lithium et de vanadium LiV₃O₈, soit un oxyde double de cobalt et de lithium optionnellement partiellement substitué de formule générale Li_{1-α}Co_{1-x+y}NiₓAl_{y} (0≤x+y≤1; 0≤y≤0,3 ; 0≤α≤1), soit un spinelle de manganèse optionnellement partiellement substitué de formule générale Li_{1-α}Mn_{2-z}M_{z} (0≤z≤1) où M = Li, Mg, Al, Cr, Ni, Co, Cu, Ni, Fe, soit un phosphate double de structure olivine ou Nasicon tels que Li_{1-α}Fe₁₋ₓMnₓPO₄, Li₁₋ₓ+2αFe₂P₁₋ₓSiₓO₄ (0≤x, α≤1), soit un sel de l'acide rhodizonique, soit un polydisulfure dérivé de l'oxydation du dimercaptoéthane, du 2,5-dimercapto-1,3,4-thiadiazole, 2,5-dimercapto-1,3,4-oxadiazole, du 1,2-dimercaptocyclobutène-3,4-dione, ou leurs mélanges.

15. Générateur électrochimique selon la revendication 11, **caractérisé en ce qu'**au moins une de ses électrodes est mélangée avec au moins une composition électrolytique selon l'une quelconque des revendications 1 à 10 et contenant au moins un sel métallique pour former une électrode composite.

16. Système de stockage de l'énergie électrique de type supercapacité, **caractérisé en ce qu'**il utilise une composition électrolytique selon l'une quelconque des revendications 1 à 10.

17. Système de stockage selon la revendication 16, **caractérisée en ce qu'**il contient, dans au moins une électrode, du carbone de grande surface spécifique.

18. Système de stockage selon la revendication 16 ou 17, **caractérisé en ce qu'**il contient dans au moins une électrode un polymère conjugué.

19. Système de stockage selon la revendication 16 ou 17, **caractérisé en ce qu'**il contient dans les deux électrodes un polymère conjugué possédant trois degrés d'oxydation.

20. Système de stockage selon la revendication 17 ou 18, **caractérisé en ce que** le polymère conjugué est un dérivé du phényl-3-thiophène.

21. Système de modulation de la lumière de type électrochrome comprenant au moins un matériau électrochrome, **caractérisé en ce qu'**il utilise une composition électrolytique suivant l'une quelconque des revendications 1 à 10..

22. Système de modulation selon la revendication 21, **caractérisé en ce que** le matériau électrochrome est déposé sur une couche d'un semi-conducteur transparent dans le visible dérivé de l'oxyde d'étain ou de l'oxyde d'indium sur un substrat de verre ou d'un polymère.

23. Système de modulation selon la revendication 21, **caractérisé en ce que** le matériau électrochrome est un oxyde de molybdène, de tungstène, de titane, de vanadium, de niobium, de cérium, d'étain ou leur mélanges.

24. Système de modulation selon l'une quelconque des revendications 21 à 23, **caractérisé en ce que** le matériau électrochrome est dissous dans l'électrolyte.

25. Utilisation d'un composé ionique comme milieu pour des réactions chimiques ou électrochimiques mettant en jeu des espèces solubles dans ledit milieu, **caractérisée en ce que** ledit composé ionique est moins un composé de bas point de fusion dont le cation est de type onium possédant au moins un hétéroatome tel que N, O, S ou P portant la charge positive et dont l'anion inclut, en totalité ou en partie, au moins un ion imidure du type (FX¹O)N⁻(OX²F) où X¹ et X² sont identiques ou différents et comprennent SO ou PF, ledit composé de bas point de fusion ayant un cation de type onium de formule : 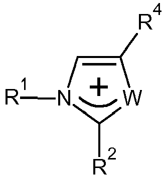 de formule 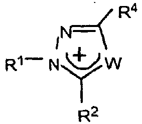 de formule 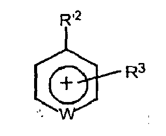 de formule 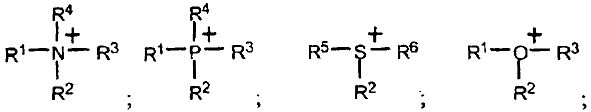 ou 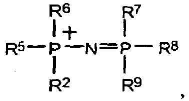 dans lesquelles
- W est O, S ou N, et dans lequel N est optionnellement substitué par R¹ lorsque la valence le permet ;
- R¹, R² et R⁴ sont identiques ou différents et représentent
- H;
- les radicaux alkyles, alkényles, oxaalkyles, oxaalkényles, azaalkyles, azaalkényles, thiaalkyles, thiaalkényles, dialkylazo, lesdits radicaux pouvant être linéaires, ramifiés ou cycliques et comprenant de 1 à 18 atomes de carbone;
- les radicaux cycliques ou hétérocycliques aliphatiques de 4 à 26 atomes de carbone comprenant optionnellement au moins une chaîne latérale comprenant un ou plusieurs hétéroatomes;
- les aryles, arylalkyles, alkylaryles et alkénylearyle de 5 à 26 atomes de carbone comprenant optionnellement un ou plusieurs hétéroatomes dans le noyau aromatique ;
- les groupes comprenant plusieurs noyaux aromatiques ou hétérocycliques, condensés ou non, contenant optionnellement un ou plusieurs atomes d'azote, d'oxygène, de soufre ou de phosphore,
deux groupements R¹, R² et R⁴ pouvant former un cycle ou un hétérocycle de 4 à 9 atomes un ou plusieurs groupements R¹, R² et R⁴ sur un même cation peuvent faire partie d'une chaîne polymère;
- et R² et R⁵ à R⁹ sont identiques ou différents et représentent, R¹O-, (R¹)₂N-, R¹S-, R¹ étant tel que défini précédemment.

26. Utilisation selon la revendication 25, comme milieu pour les réactions de Diels-Alder, de Friedel-Craft, d'aldolisation de condensation, de polymérisation anionique, cationique ou radicalaire, et pour les substitutions nucléophiles et électrophiles.

27. Utilisation selon la revendication 25 comme solvant chiral dans une réaction chimique pour la formation d'excès énantiomériques, **caractérisé en ce que** le cation du composé à bas point de fusion est un cation ammonium, sulfonium ou phosphonium.

28. Utilisation selon la revendication 25, caractérisé en ce le milieu contient au moins une espèce catalytique.

29. Utilisation selon la revendication 28 **caractérisé en ce que** l'espèce catalytique est au moins un sel de transition et/ou un sel de terre rare et/ou un sel organométallique tel que les métallocènes, les sels métalliques pouvant être coordonnés avec les ligands.

30. Utilisation selon la revendication 29 **caractérisé en ce que** les ligands sont choisis dans le groupe constitué par les bipyridines, porphyrines, phosphines, et les arsines.

## Patentansprüche

1. Elektrolytzusammensetzung, umfassend mindestens eine Verbindung mit niedrigem Schmelzpunkt, deren Kation vom Typ Onium ist und mindestens ein Heteroatom wie N, O, S oder P besitzt, das die positive Ladung trägt, und deren Anion insgesamt oder teilweise mindestens ein Imidion vom Typ (FX¹O)N⁻(OX²F) einschließt, wobei X¹ und X² identisch oder unterschiedlich sind und SO oder PF umfassen, wobei die Zusammensetzung mindestens einen anderen Bestandteil enthält, der ein Metallsalz, ein polares Polymer und/oder ein aprotisches Co-Solvens umfasst, wobei die Elektrolytzusammensetzung **dadurch gekennzeichnet ist, dass** die Verbindung mit niedrigem Schmelzpunkt ein Kation vom Typ Onium der Formel: 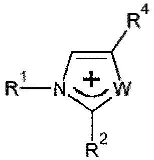 der Formel 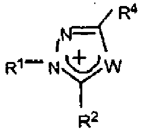 der Formel 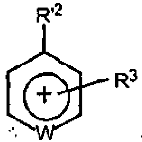 der Formel 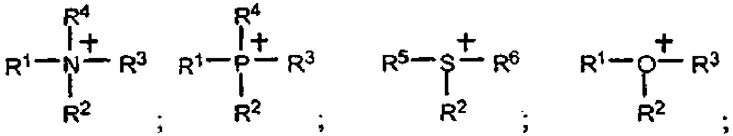 oder 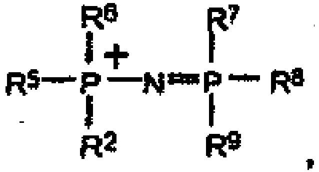 hat, wobei
- W O, S oder N ist, und wobei N optional durch R¹ substituiert ist, wenn die Wertigkeit dies erlaubt,
- R¹, R³, R⁴ identisch oder unterschiedlich sind und darstellen
- H,
- die Alkyl-, Alkenyl-, Oxaalkyl-, Oxaalkenyl-, Azaalkyl-, Azaalkenyl-, Thiaalkyl-, Thiaalkenyl-, Dialkylazo-Radikale, wobei die Radikale linear, verzweigt oder zyklisch sein können und 1 bis 18 Kohlenstoffatome umfassen,
- die zyklischen oder heterozyklischen aliphatischen Radikale mit 4 bis 26 Kohlenstoffatomen, die optional mindestens eine Seitenkette umfassen, die ein oder mehrere Heteroatome umfasst,
- die Aryle, Arylalkyle, Alkylaryle und Alkenylaryle mit 5 bis 26 Kohlenstoffatomen, die optional ein oder mehrere Heteroatome im aromatischen Kern umfassen,
- die Gruppen, die mehrere aromatische oder heterozyklische Kerne, kondensiert oder nicht, umfassen, die optional ein oder mehrere Stickstoff-, Sauerstoff-, Schwefel- oder Phosphoratome enthalten,
wobei zwei Gruppen R¹, R³ oder R⁴ einen Ring oder einen Heterocyclus mit 4 bis 9 Atomen bilden können, wobei eine oder mehrere Gruppen R¹, R³ oder R⁴ auf einem selben Kation Bestandteil einer Polymerkette sein können,
- und R² und R⁵ bis R⁹ identisch oder unterschiedlich sind und R¹, R¹O-, (R¹)₂N-, R¹S- darstellen, wobei R¹ wie zuvor definiert ist.

2. Elektrolytzusammensetzung nach Anspruch 1, wobei die Gruppen R¹, R³ und R⁴ eine polymerisationsaktive Gruppe tragen können.

3. Elektrolytzusammensetzung nach Anspruch 2, wobei die polymerisationsaktive Gruppe die Doppelbindungen, die Epoxyde oder die in den Polykondensationen reaktiven Funktionen umfasst.

4. Elektrolytzusammensetzung nach Anspruch 1, wobei das Kation ein Ammonium-, Imidazolium-, Pyridinium- oder Phosphoniumion, nicht substituiert oder substituiert durch eine Alkyl-, Oxaalkyl- oder Dialkylamino-Gruppe, umfasst, die 1 bis 8 Kohlenstoffatome umfasst.

5. Elektrolytzusammensetzung nach einem der Ansprüche 1 bis 4, die ferner mindestens ein Anion enthält, das aus Cl, Br-; I-; NO₃⁻; M(R¹⁰)₄⁻, A(R¹⁰)₆⁻_{;} R¹¹O₂⁻, [R¹¹ONZ¹]⁻, [R¹¹YOCZ²Z³]⁻, 4,5-Dicyano-1,2,3-triazol, 3,5-bis(Rf)-1,2,4-triazol, Tricyanomethan, Pentacyanocyclopentadien, Pentakis(trifluormethyl)cyclopentadien, den Derivaten der Barbitursäure und der Meldrumsäure und ihren Substitutionsprodukten ausgewählt ist, wobei
- M B, Al, Ga oder Bi ist,
- A P, As und Sb ist,
- R¹⁰ ein Halogen ist,
- R¹¹ H, F, eine Alkyl-, Alkenyl-, Aryl-, Arylalkyl-, Alkylaryl-, Arylalkenyl-, Alkenylaryl-, Dialkylamino-, Alcoxy- oder Thioalcoxy-Gruppe darstellt, wobei jede 1 bis 18 Kohlenstoffatome hat und von einem oder mehreren Oxa-, Thia- oder Aza-Substituenten nicht substituiert oder substituiert ist, und wobei ein oder mehreren Wasserstoffatome durch ein Halogen in einem Verhältnis von 0 bis 100 % ersetzt sind und eventuell Teil einer Polymerkette sein können,
- Y C, SO, S=NCN, S=C(CN)₂, POR¹¹, P(NCN)R¹¹, P(C(CN)₂R¹¹, eine Alkyl-, Alkenyl-, Aryl-, Arylalkyl-, Alkylaryl-, Arylalkenyl-, Alkenylaryl-Gruppe, die 1 bis 18 Kohlenstoffatome besitzt und optional von einem oder mehreren Oxa-, Thia- oder Aza-Substituenten substituiert ist, eine Dialkylamino-Gruppe N(R¹⁰)₂ darstellt,
- Z¹ bis Z³ unabhängig R¹¹, R¹¹YO oder CN darstellen, wobei diese Gruppe optional Bestandteil einer Polymerkette sein kann.

6. Elektrolytzusammensetzung nach Anspruch 1, **dadurch gekennzeichnet, dass** das Kation des Metallsalzes ein Kation eines alkalischen Metalls, eines erdalkalischen Metalls, eines Übergangsmetalls und einer seltenen Erde ist.

7. Elektrolytzusammensetzung nach Anspruch 6, **dadurch gekennzeichnet, dass** mindestens ein Metallsalz ein Lithiumsalz ist.

8. Elektrolytzusammensetzung nach Anspruch 1, **dadurch gekennzeichnet, dass** das polare Polymer Monomereinheiten umfasst, die Derivate von Ethylenoxid, Propylenoxid, Epichlorhydrin, Epifluorhydrin, Trifluorepoxypropan, Acrylnitril, Methacrylnitril, Ester und Amide der Acryl- und Methacrylsäure, Vinylidenfluorid, N-Methylpyrolidon und Polyelektrolyte vom Typ Polykation oder Polyanion sind.

9. Elektrolytzusammensetzung nach Anspruch 8, **dadurch gekennzeichnet, dass** mindestens eines der Polymere vernetzt ist, wenn die Zusammensetzung mehr als ein Polymer umfasst.

10. Elektrolytzusammensetzung nach Anspruch 1, **dadurch gekennzeichnet, dass** das aprotische Co-Solvens aus den Dialkylethern des Ethylenglycols, des Diethylenglycols, des Triethylenglycols, der Polyethylenglycole mit einer Masse zwischen 400 und 2000 inklusive; den Estern, insbesondere denen der Kohlensäure, linearen oder cyclischen, wie Dimethylcarbonat, Methyl-ethylcarbonat, Ethylencarbonat, Propylencarbonat; den Estern wie -Butyrolacton, den Nitrilen wie Glutaronitril, 1,2,6-Tricyanohexan, den Amiden wie Dimethylformamid, N-Methylpyrrolidinon, den Sulfamiden und Sulfonamiden sowie den Gemischen der vorgenannten Verbindungen ausgewählt ist.

11. Elektrochemischer Generator mit mindestens einer positiven Elektrode und mindestens einer negativen Elektrode, **dadurch gekennzeichnet, dass** er eine Elektrolytzusammensetzung nach einem der Ansprüche 1 bis 10 verwendet.

12. Elektrochemischer Generator nach Anspruch 11, **dadurch gekennzeichnet, dass** die Elektrolytzusammensetzung mindestens ein Lithiumsalz enthält.

13. Elektrochemischer Generator nach Anspruch 11 oder 12, **dadurch gekennzeichnet, dass** die negative Elektrode entweder metallisches Lithium oder eine seiner Legierungen oder ein Insertionsverbindung des Kohlenstoffs, insbesondere Petrol- oder Graphitkoks, oder ein Oxid mit niedrigem Insertionspotential wie die Titanspinelle Li₄₋ₓ₊₃yTi₅₋ₓOₗ₂ (0≤x, y≤1), oder ein Doppelnitrid eines Übergangsmetalls und von Lithium wie Li₃₋ₓCo_{z}N (0≤z≤1) oder mit der Struktur vom Typ Antifluorid wie Li₃FeN₂ oder Li₇MnN₄ oder eines ihrer Gemische enthält.

14. Elektrochemischer Generator nach Anspruch 11 oder 12, **dadurch gekennzeichnet, dass** die positive Elektrode entweder Vanadiumoxid VOₓ (2≤x≤2,5) oder Lithium- und Vanadium-Mischoxid LiV₃O₈ oder Kobalt- und Lithium-Doppeloxid, optional teilweise substituiert, der allgemeinen Formel Li_{1-α}Co_{1-x+y}NiₓAl_{y}(0≤x+y≤1; 0≤y≤0,3, 0≤α≤1) oder ein Manganspinell, optional teilweise substituiert, der allgemeinen Formel Li_{1-α}Mn_{2-z}M_{z} (0≤z≤1), wobei M = Li, Mg, Al, Cr, Ni, Co, Cu, Ni, Fe, oder ein Phosphat mit doppelter Olivin- oder Nasiconstruktur wie Li_{1-α}Fe₁₋ₓMnₓPO₄, Li₁₋ₓ+2αFe₂P₁₋ₓSiₓO₄ (0≤x, α≤1) oder ein Salz der Rhodizonsäure oder ein Polydisulfid als Derivat der Oxidation von Dimercaptoethan, 2,5-Dimercapto-1,3,4-thiadiazol, 2,5-Dimercapto-1,3,4-oxadiazol, 1,2-Dimercaptocyclobuten-3,4-dion oder ihre Gemische enthält.

15. Elektrochemischer Generator nach Anspruch 11, **dadurch gekennzeichnet, dass** mindestens eine seiner Elektroden mit mindestens einer Elektrolytzusammensetzung nach einem der Ansprüche 1 bis 10 gemischt ist und mindestens ein Metallsalz enthält, um eine Verbundelektrode zu bilden.

16. Speichersystem elektrischer Energie vom Typ Superkapazität, **dadurch gekennzeichnet, dass** es eine Elektrolytzusammensetzung nach einem der Ansprüche 1 bis 10 verwendet.

17. Speichersystem nach Anspruch 16, **dadurch gekennzeichnet, dass** es in mindestens einer Elektrode Kohlenstoff mit großer spezifischer Oberfläche enthält.

18. Speichersystem nach Anspruch 16 oder 17, **dadurch gekennzeichnet, dass** es in mindestens einer Elektrode ein konjugiertes Polymer enthält.

19. Speichersystem nach Anspruch 16 oder 17, **dadurch gekennzeichnet, dass** es in den zwei Elektroden ein konjugiertes Polymer enthält, das drei Oxidationsgrade besitzt.

20. Speichersystem nach Anspruch 17 oder 18, **dadurch gekennzeichnet, dass** das konjugierte Polymer ein Derivat von Phenyl-3-thiophen ist.

21. Modulationssystem des Lichts vom Typ elektrochrom, umfassend mindestens ein elektrochromes Material, **dadurch gekennzeichnet, dass** es eine Elektrolytzusammensetzung nach einem der Ansprüche 1 bis 10 verwendet.

22. Modulationssystem nach Anspruch 21, **dadurch gekennzeichnet, dass** das elektrochrome Material auf einer im Sichtbaren transparenten Schicht eines Halbleiters, abgeleitet von Zinnoxid oder von Indiumoxid auf einem Glas- oder einem Polymersubstrat, aufgebracht ist.

23. Modulationssystem nach Anspruch 21, **dadurch gekennzeichnet, dass** das elektrochrome Material ein Oxid von Molybden, Wolfram, Titan, Vanadium, Niobium, Cerium, Zinn oder ihrer Gemische ist.

24. Modulationssystem nach einem der Ansprüche 21 bis 23, **dadurch gekennzeichnet, dass** das elektrochrome Material in dem Elektrolyt gelöst ist.

25. Verwendung einer lonenverbindung als Medium für chemische oder elektrochemische Reaktionen, bei denen in dem Medium lösliche Spezies zum Einsatz kommen, **dadurch gekennzeichnet, dass** die Ionenverbindung mindestens eine Verbindung mit niedrigem Schmelzpunkt ist, deren Kation vom Typ Onium ist und mindestens ein Heteroatom wie N, O, S oder P besitzt, das die positive Ladung trägt, und deren Anion insgesamt oder teilweise mindestens ein Imidion vom Typ (FX¹O)N⁻(OX²F) einschließt, wobei X¹ und X² identisch oder unterschiedlich sind und SO oder PF umfassen, wobei die Verbindung mit niedrigem Schmelzpunkt ein Kation vom Typ Onium der Formel:. 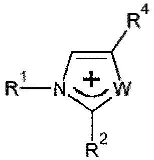 der Formel 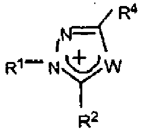 der Formel 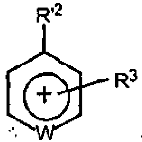 der Formel 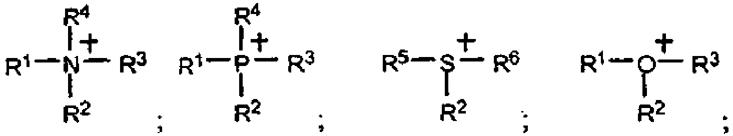 oder 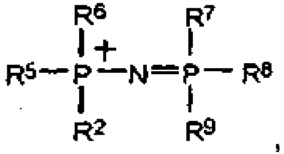 hat, wobei
- W O, S oder N ist, und wobei N optional durch R¹ substituiert ist, wenn die Wertigkeit dies erlaubt,
- R¹, R² und R⁴ identisch oder unterschiedlich sind und darstellen
- H,
- die Alkyl-, Alkenyl-, Oxaalkyl-, Oxaalkenyl-, Azaalkyl-, Azaalkenyl-, Thiaalkyl-, Thiaalkenyl-, Dialkylazo-Radikale, wobei die Radikale linear, verzweigt oder zyklisch sein können und 1 bis 18 Kohlenstoffatome umfassen,
- die zyklischen oder heterozyklischen aliphatischen Radikale mit 4 bis 26 Kohlenstoffatomen, die optional mindestens eine Seitenkette umfassen, die ein oder mehrere Heteroatome umfasst,
- die Aryle, Arylalkyle, Alkylaryle und Alkenylaryle mit 5 bis 26 Kohlenstoffatomen, die optional ein oder mehrere Heteroatome im aromatischen Kern umfassen,
- die Gruppen, die mehrere aromatische oder heterozyklische Kerne, kondensiert oder nicht, umfassen, die optional ein oder mehrere Stickstoff-, Sauerstoff-, Schwefel- oder Phosphoratome enthalten,
wobei zwei Gruppen R¹, R² und R⁴ einen Ring oder einen Heterocyclus mit 4 bis 9 Atomen bilden können, wobei eine oder mehrere Gruppen R¹, R² und R⁴ auf einem selben Kation Bestandteil einer Polymerkette sein können,
- und R² und R⁵ bis R⁹ identisch oder unterschiedlich sind und R¹O-, (R¹)₂N-, R¹S- darstellen, wobei R¹ wie zuvor definiert ist.

26. Verwendung nach Anspruch 25 als Medium für die Diels-Alder-, Friedel-Craft-, Kondensations-Aldolisations-, anionische, kationische oder Radikal-Polymerisations-Reaktionen und für die nukleophilen und elektrophilen Substitutionen.

27. Verwendung nach Anspruch 25 als chirales Lösungsmittel in einer chemischen Reaktion für die Bildung von Enantiomerenüberschüssen, **dadurch gekennzeichnet, dass** das Kation der Verbindung mit niedrigem Schmelzpunkt ein Ammonium-, Sulfonium- oder Phosphonium-Kation ist.

28. Verwendung nach Anspruch 25, **dadurch gekennzeichnet, dass** das Medium mindestens eine katalytische Spezies enthält.

29. Verwendung nach Anspruch 28, **dadurch gekennzeichnet, dass** die katalytische Spezies mindestens ein Übergangssalz und/oder ein Seltene-Erden-Salz und/oder ein organometallisches Salz wie die Metallocene ist, wobei die Metallsalze mit den Liganden koordiniert sein können.

30. Verwendung nach Anspruch 29, **dadurch gekennzeichnet, dass** die Liganden aus der Gruppe ausgewählt sind, die von den Bipyridinen, Prophyrinen, Phosphinen und den Arsinen gebildet ist.

## Claims

1. An electrolytic composition comprising at least one low melting-point compound, the cation of which is of the onium type having at least one heteroatom such as N, 0, S or P bearing the positive charge and the anion of which totally or partly includes at least one imide ion of the type (FX¹O) N⁻ (OX²F) wherein X¹ and X² are identical or different and comprise SO or PF, said composition containing at least one other component comprising a metal salt, a polar polymer and/or an aprotic solvent, said electrolytic composition being **characterized by** the fact that said low melting-point compound has a cation of the onium type of formula: 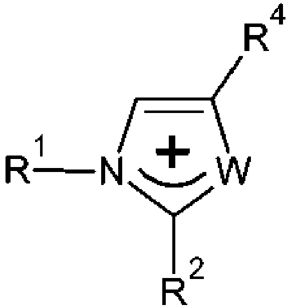 of formula 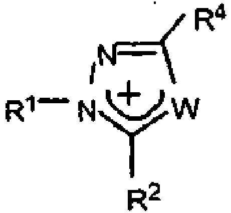 of formula 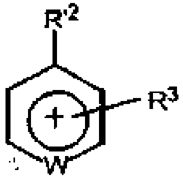 of formula 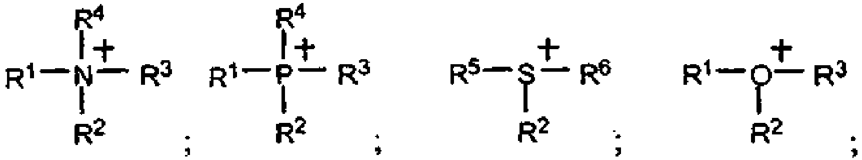 or 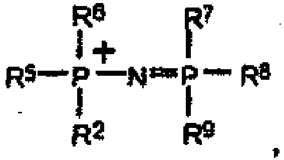 wherein
- W is 0, S or N, and wherein N is optionally substituted with R¹ when valency allows this;
- R¹, R³ and R⁴ are either identical or different and represent:
- H;
- alkyl, alkenyl, oxaalkyl, oxaalkenyl, azaalkyl, azaalkenyl, thiaalkyl, thiaalkenyl, dialkylazo radicals, said radicals may be linear, branched or cyclic and comprising from 1 to 18 carbon atoms;
- aliphatic cyclic or heterocyclic radicals from 4 to 26 carbon atoms optionally comprising at least one side chain comprising one or several heteroatoms;
- aryls, arylalkyls, alkylaryls, and alkenylaryls from 5 to 26 carbon atoms optionally comprising one or several heteroatoms in the aromatic ring;
- groups comprising several aromatic or heterocyclic rings either fused or not, optionally comprising one or several nitrogen, oxygen, sulfur or phosphorus atoms,
two groups R¹, R³ or R⁴ may form a ring or a heterocycle from 4 to 9 atoms, one or several groups R¹, R³ or R⁴ on a same cation may be part of a polymer chain;
- and R2 and R5 to R9 are either identical or different and represent R¹, R¹O-, (R¹) ₂N-, R¹S-, R¹ being as defined previously.

2. The electrolytic composition according to claim 1, wherein the groups R¹, R³ and R⁴ may bear an active group in polymerization.

3. The electrolytic composition according to claim 2, wherein the active group in polymerization comprises double bonds, epoxides, or reactive functions in polycondensation.

4. The electrolytic composition according to claim 1, wherein the cation comprises an ammonium, imidazolium, pyridinium or phosphonium ion, either non-substituted or substituted with an alkyl, oxaalkyl or dialkylamino group comprising from 1 to 8 carbon atoms.

5. The electrolytic composition according to any of claims 1 to 4, which further contains at least one anion selected from among Cl⁻; Br⁻; I⁻ ; NO₃⁻ M(R¹⁰)₄⁻ , A(R¹⁰)₆⁻ , R¹¹O₂⁻ , [R¹¹ONZ¹]⁻, [R¹¹YOCZ²Z³ ]⁻, 4,5-dicyano-1,2,3-triazole, 3, 5bis (R_{f})-1,2,4-triazole, tricyanomethane, pentacyanocyclopentadiene, pentakis(trifluoromethyl)cyclopentadiene, derivatives of barbituric acid and Meldrum acid and substitution products thereof;
- M is B, Al, Ga or Bi;
- A is P, As and Sb;
- R¹⁰ is a halogen;
- R¹¹ representing H, F, an alkyl, alkenyl, aryl, arylalkyl, alkylaryl, arylalkenyl, alkenylaryl, dialkylamino, alkoxy or thioalkoxy group, each having from 1 to 18 carbon atoms and being substituted or non-substituted with one or several oxa, thia or aza substituents, and wherein on or several hydrogen atoms are optionally replaced with a halogen in a proportion from o to 100% and may optionally be part of a polymer chain;
- Y representing C, SO, S=NCN, S=C(CN)₂, POR¹¹, P(NCN)R¹¹, P(C(CN)₂)R¹¹, an alkyl, alkenyl, aryl, arylalkyl, alkylaryl, arylalkenyl, alkenylaryl group having from 1 to 18 carbon atoms and optionally substituted with one or several oxa, thia or aza substituents; a dialkyamino-N(R¹⁰)₂ group;
- Z1 to Z3 represent independently R¹¹, R¹¹YO or CN, this group may optionally be part of a polymer chain.

6. The electrolytic composition according to claim 1, **characterized in that** the metal salt cation is a cation of an alkaline metal, an earth alkaline metal, a transition metal and a rare earth.

7. The electrolytic composition according to claim 6, **characterized in that** at least one metal salt is a lithium salt.

8. The electrolytic composition according to claim 1, **characterized in that** the polar polymer comprises monomer units derived from ethylene oxide, propylene oxide, epichlorohydrin, epifluorohydrin, trifluoroepoxypropane, acrylonitrile, methacrylonitrile, esters and amides of acrylic and methacrylic acid, vinylidene fluoride, N-methylpyrrolidone and polyelectrolytes of the polyation or polyanion type.

9. The electrolytic composition according to claim 8, **characterized in that** at least one of the polymers is crosslinked.

10. The electrolytic composition according to claim 1, **characterized in that** the aprotic co-solvent is selected from dialkylethers of ethylene glycol, propylene glycol, triethylene glycol, polyethylene glycols with a mass comprised between 400 and 2000; linear or cyclic esters, in particular those of carbonic acid, such as dimethyl carbonate, methylethyl carbonate, diethyl carbonate, ethylene carbonate, propylene carbonate; esters like γ-butyrolactone, nitriles like glutaronitrile, 1,2,6-tricyanohexane; amides like dimethylformamide, N-methylpyrrolidinone, sulfamides and sulfonamides as well as mixtures of the aforementioned compounds.

11. An electrochemical generator having at least one positive electrode and at least one negative electrode, **characterized in that** it uses an electrolytic composition according to any of claims 1 to 10.

12. The electrochemical generator according to claim 11, **characterized in that** said electrolytic composition contains at least one lithium salt.

13. The electrochemical generator according to claim 11 or 12, **characterized in that** the negative electrode either contains lithium metal or one of its alloys or a carbon insertion compound, in particular, petroleum coke or graphite, or an oxide with low insertion potential such as titanium spinelles Li_{4-x+3y}Ti₅₋ₓO₁₂ (0≤x, y≤1), or a double nitride of a transition metal and of lithium like Li₃₋ₓCo_{z}N (0≤z≤1) or having the anti-fluorite type structure like Li₃FeN₂ or Li₇MnN₄, or mixtures thereof.

14. The electrochemical generator according to claim 11 or 12, **characterized in that** the positive electrode either contains vanadium oxide VOₓ (2≤x≤2.5) or a mixed lithium and vanadium oxide LiV₃O₈, or a double cobalt and lithium oxide optionally partly substituted of general formula Li_{1-α}Co_{1-x+y}NiₓAl_{y}, (0≤x+y≤1); (0≤y≤0.3); (0≤α≤1) , i.e. a manganese spinelle optionally partly substitutedof general formula Li_{1-α}Mn_{2-z}M_{z}(0≤z≤1) wherein M = Li, Mg, Al, Cr, Ni, Co, Cu, Ni, Fe, or a double phosphate with an olivine structure or Nasicon such as Li_{1-α}Fe₁₋ₓMnₓPO₄, Li₁₋ₓ+2αFe₂P₁₋ₓSiₓO₄ (0≤x,α≤1), or a salt of rhodizonic acid, or a polydisulfide derived from the oxidation of dimercaptoethane, 2,5-dimercapto-1,3,4-thiadiazole, 2,5-dimercapto-1,3,4-oxadiazole, 1,2-dimercaptocyclobutene-3,4-dione or mixtures thereof.

15. The electrochemical generator according to claim 11, **characterized in that** at least one of its electrodes is mixed with at least one electrolytic composition according to any of claims 1 to 10 and containing at least one metal salt in order to form a composite electrode.

16. A system for storing electric energy of the supercapacitor type, **characterized in that** it uses an electrolytic composition according to any of claims 1 to 10.

17. The storage system according to claim 16, **characterized in that** it contains, in at least one electrode, carbon with a large specific surface area.

18. The storage system according to claim 16 or 17, **characterized in that** it contains, in at least one electrode, a conjugate polymer.

19. The storage system according to claim 16 or 17, **characterized in that** it contains in both electrodes a conjugate polymer having three degrees of oxidation.

20. The storage system according to claim 17 or 18, **characterized in that** the conjugate polymer is a derivative of phenyl-thiophene.

21. A light modulation system of the electrochromic type comprising at least one electrochromic material, **characterized in that** it uses an electrolytic composition according to any of claims 1 to 10.

22. The modulation system according to claim 21, **characterized in that** the electrochromic material is deposited on a layer of a semiconductor transparent in visible light, derived from tin oxide or indium oxide on a substrate of glass or of a polymer.

23. The modulation system according to claim 21, **characterized in that** the electrochromic material is an oxide of molybdenum, tungsten, titanium, vanadium, niobium, cerium, tin or mixtures thereof.

24. The modulation system according to any of claims 21 to 23, **characterized in that** the electrochromic material is dissolved in the electrolyte.

25. The use of an ion compound as a medium for chemical or electrochemical reactions setting into play soluble species in said medium, **characterized in that** said ion compound is at least one low melting-point compound, the cation of which is of the onium type having at least one heteroatom such as N, 0, S or P, bearing the positive charge and the anion of which totally or partly includes at least one imide ion of the type (FX¹O)N⁻ (OX²F) wherein X¹ and X² are identical or different and comprise SO or PF, said low melting-point compound having a cation of the onium type of formula: 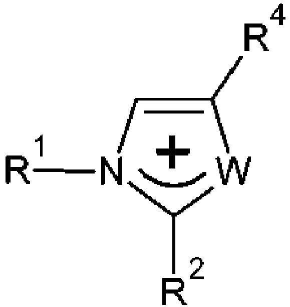 of formula 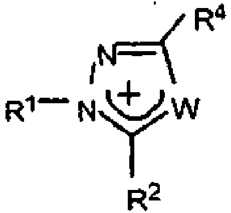 of formula 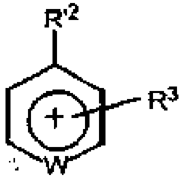 of formula 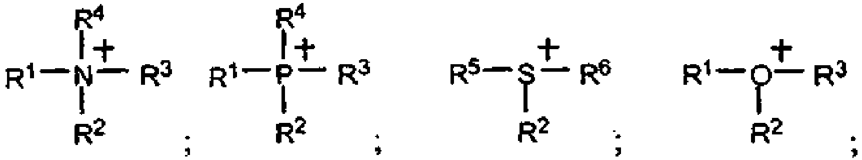 or 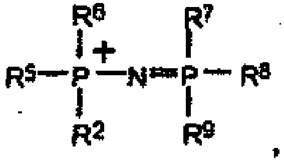 wherein
- W is 0, S or N, and wherein N is optionally substituted with R¹ when valency allows this;
- R¹, R² and R⁴ are either identical or different and represent:
- H;
- alkyl, alkenyl, oxaalkyl, oxaalkenyl, azaalkyl, azaalkenyl, thiaalkyl, thiaalkenyl, dialkylazo radicals, said radicals may be linear, branched or cyclic and comprising from 1 to 18 carbon atoms;
- aliphatic cyclic or heterocyclic radicals from 4 to 26 carbon atoms optionally comprising at least one side chain comprising one or several heteroatoms;
- aryls, arylalkyls, alkylaryls, and alkenylaryls from 5 to 26 carbon atoms optionally comprising one or several heteroatoms in the aromatic ring;
- groups comprising several aromatic or heterocyclic rings either fused or not, optionally comprising one or several nitrogen, oxygen, sulfur or phosphorus atoms,
two groups R¹, R² or R⁴ may form a ring or a heterocycle from 4 to 9 atoms, one or several groups R¹, R² or R⁴ on a same cation may be part of a polymer chain;
- and R² and R⁵ to R⁹ are either identical or different and represent R¹O-, (R¹)₂N-, R¹S-, R¹ being as defined previously.

26. The use according to claim 25, as a medium for Diels-Alder, Friedel-Craft, condensation aldolization, anionic, cationic or radical polymerization reactions, and for nucleophilic and electrophilic substitutions.

27. The use according to claim 25, as a chiral solvent in a chemical reaction for forming enantiomeric excesses, **characterized in that** the cation of the low melting-point compound is an ammonium, sulfonium or phosphonium cation.

28. The use according to claim 25, **characterized in that** the medium contains at least one catalytic species.

29. The use according to claim 28, **characterized in that** the catalytic species is at least one transition salt and/or rare earth salt and/or organometallic salt such as metallocenes, the metal salts may be coordinated with ligands.

30. The use according to claim 29, **characterized in that** the ligands are selected from the group formed by pyridines, porphyrines, phosphines and arsines.
